# EUROPEAN PATENT SPECIFICATION

(11) **EP 2 701 688 B1**
(45) Date of publication and mention of the grant of the patent: **14.09.2016**
(21) Application number: 12718174.1
(22) Date of filing: 25.04.2012
(51) Int. Cl.: A61K 36/16, A61K 9/20

(54) **CONTROLLED RELEASE TABLET OF GINKGO BILOBA EXTRACT AND PROCEDURE FOR OBTAINING IT**
RETARD-TABLETTE MIT GINKGO-BILOBA-EXTRAKT UND VERFAHREN ZU IHRER HERSTELLUNG
COMPRIMÉ À LIBÉRATION CONTRÔLÉE D'UN EXTRAIT DE GINKGO BILOBA ET PROCÉDÉ POUR SON OBTENTION

(30) Priority: 27.04.2011 MX 2011004417
(43) Date of publication of application: 05.03.2014
(73) Proprietor: Dr. Willmar Schwabe GmbH & Co. KG, 76227 Karlsruhe (DE)
(72) Inventor: HERRMANN, Joachim, 68789 St. Leon-Rot (DE); JECHLITSCHKA, Sven, Robert, 03100 México D.F. (MX); ROTHE, Andreas, 76227 Karlsruhe (DE); THÖLE, Marc, 76227 Karlsruhe (DE)
(74) Representative: Adam, Holger
(86) International application number: PCT/EP2012/057506
(87) International publication number: WO 2012/146592

(56) References cited:
- CN-A- 101 011 381
- US-A1- 2008 171 085
- Rite Care Pharmacy: "Vitaline Ginkgo Biloba Plus", , 5 May 2009 (2009-05-05), XP002683501, Retrieved from the Internet: URL:http://www.ritecare.com/prodsheets/VTF -324003.html [retrieved on 2012-09-12]
- ratiopharm: "Gebrauchsinformation Ginkobil ratiopharm 120mg", , 30 September 2008 (2008-09-30), XP002683502, Retrieved from the Internet: URL:http://www.arzneicom.de/productimages/ hashed/6/6/8/6680881p.pdf [retrieved on 2012-09-14]

## Description

### Field of the invention

The present invention as defined by the claims refers to pharmaceutical formulations with controlled release of the active principle and more particularly, it refers to controlled release tablets of ginkgo biloba extract and a novel procedure for preparing controlled release tablets of herbal extracts.

### Background of the invention

Pharmaceutical dosage forms can display different release profiles for the active ingredients which are classified by the European Pharmacopeia Chapter 2.9.3 ("Dissolution of solid dosage forms") as "immediate release" (75 % of the active ingredient is released from the dosage form within 45 minutes), "prolonged release" (describing the release profile over an extended time period with three time points) and "delayed release".

In this invention, the expression "controlled release" is used to describe the release profile of the dosage being controlled over an extended period of time. As it is known, the advantages of controlled release dosage forms can be, for example: better therapeutic action, fewer side effects (as concentration peaks of the active in the bloodstream are avoided), the optimum drug level is maintained in the bloodstream during a longer time period, the application frequency is reduced; however, there are some disadvantages such as higher production costs, frequently less or different bioavailability, risks associated with the possibility of immediate release of the entire dose and a greater risk associated with the possibility of developing tolerance to the active component.

With respect to controlled release drug preparations, the release modifying principle which is used to achieve a controlled drug release can be classified as shown below [H.-W. Hui, J.R. Robinson: Design and fabrication of oral controlled delivery drug delivery systems. In: Controlled Drug Delivery, Marcel Dekker, 1987, p. 373]
a) Dissolution controlled release
b) Diffusion controlled release
c) Diffusion and dissolution controlled release
d) Ion-exchange resins
e) pH dependent formulations
f) Osmoctically controlled release
g) Altered density formulations

In many cases, it is not possible to define a single release mechanism for a drug; instead there is a combination of several of these factors. The system of the present invention may be classified as a diffusion and dissolution controlled release device.

### Plant extracts or herbal medicines

As it is known, plant extracts or herbal medicines do not contain one active substance, but are rather preparations obtained from plants which represent a mixture of several chemically different substances. Many times it is impossible to determine the "active principle" itself, that is, to detect the fraction of ingredients which actually produces the therapeutic effect. Besides, it can be very difficult to chemically capture them, be it a group of molecules or one substance. Besides this, there may be a poor stability of herbal preparations, since in their natural composition they contain secondary substances which can easily undergo physicochemical changes and affect the organoleptic quality of the drug preparation (color, smell, taste) and/or its physicochemical properties (content, disintegration time, hardness, release profile). In many cases, the fraction of the active principle has high sensitivity to environmental factors such as humidity, heat, light and oxygen.

Due to this complexity of the active principle in plant extracts, it is difficult to develop a stable herbal medicine, even more difficult as a controlled release pharmaceutical form.

The document CN101011381 discloses sustained release floating tablets comprising Ginkgo leaf extract (about 75 wt %), in a matrix composition of approx. 25 wt% excipients like HPC, CMC and ethylcellulose and calcium carbonate.

From all the above, it is necessary to design procedures for the development of plant extracts dosage forms with the appropriate quality and stability and with controlled release of the active principle from the dosage form. A number of products (mostly food supplements) can be found in the market, advertised as innovative and, claiming effects without scientific substantiation, often in combination with suffixes in their brand name as "CR" or "extended release" or "with prolonged effect".

The applicant (Dr. Willmar Schwabe GmbH & Co. KG) in the past has played an important role as an innovator and leader in the development of herbal medicinal products, and most prominently in the therapeutic use of Ginkgo biloba and special extracts obtained from this plant. It is known that Ginkgo biloba is a powerful artery vasodilator and it is used for the treatment of hypoxia, particularly cerebral hypoxia, besides stabilizing the veins in case of their dilation (varicose veins).

The object of the present invention is to provide an oral delivery system as defined by the claims controlling the dissolution and release of Ginkgo leaf extract in a way that avoids the disadvantages of conventional immediate release drug dosage forms such as short therapeutic action and highly fluctuating plasma levels of the active ingredients.

### Brief description of the invention

Basically, the invention consists of a tablet as defined by the claims with a controlled release profile of the active ingredient, containing dry extract from the leaves of Ginkgo biloba in a polymeric matrix of ethylcellulose, produced by compression of the dry extract from the leaves of Ginkgo biloba together with ethylcellulose and silicon dioxide, and further containing magnesium stearate or stearic acid. The release profile can be optimized and controlled through the variation of the proportions of the different ingredients.

### Detailed description of the invention and preferred embodiment

The following is a detailed description of the invention and a preferred embodiment, provided solely as a non-limiting example thereof, since in view of the description, different embodiments are possible in the components, in their dimensions, forms or materials, without contemplating another invention since this invention must be seen in its widest sense, including all that which is analogous as regards its use, known or to be known.

Controlled release in terms of the present invention means prolonged release (preferably) or delayed release according to Ph. Eur. 6.0, 2.9.3.

In a preferred embodiment a dry extract from the leaves of Ginkgo biloba is used, containing
- 20 to 30 % by weight flavonoids, selected from the group comprising quercetin, kaempferol and isorhamnetin glycosides,
- 4.5 to 8.5 % by weight terpene lactones and
- less than 10 ppm ginkgolic acids.

In a further preferred embodiment a dry extract from the leaves of Ginkgo biloba according to the European Pharmacopeia (Ph. Eur. 6.1) (e.g. the extract produced by Schwabe called EGb761^{®}) is used, containing
- 22.0 to 27.0 % by weight flavonoids, selected from the group comprising quercetin, kaempferol and isorhamnetin glycosides,
- 2.8 to 3.4 % by weight ginkgolides A, B and C in total,
- 2.6 to 3.2 % by weight bilobalid and
- 5 ppm ginkgolic acids at the most.

The extract EGb761^{®} is obtained with different steps of selective extraction, separation and purification, which enable to modify the profile of the different groups of components in such a way that the desired substances are accumulated and the content of the substances which do not contribute to the therapeutic effect, causing undesired effects or disturbing with respect to the extract stability, are reduced or eliminated, obtaining a product with a better therapeutic profile. Typically, EGb761^{®} can be prepared by the method given in EP431535B1. EGb761^{®} is already known in the market.

In a further preferred embodiment the dry extract from the leaves of Ginkgo biloba contains
- less than 10 ppm 4'-O-methyl pyridoxine and/or
- less than 20 ppm biflavones selected from the group comprising amentoflavone, bilobetin, ginkgetin, isoginkgetin and sciadopitysin.

The production of this extract is described in EP 1868625 B1.

Further disclosed in a tablet formulation that contains a mixture of 45% - 68% dry Ginkgo biloba extract (preferably EGb761^{®}), 30% - 52% ethylcellulose, 0.4% - 1.0% colloidal silicon dioxide and 1.5% - 2.5% magnesium stearate or stearic acid.

A further preferred tablet formulation according to the present invention contains a mixture of 52% - 60% dry Ginkgo biloba extract (preferably EGb761^{®}), 37% - 45% ethylcellulose, 0.4% - 1.0% colloidal silicon dioxide and 1.5% - 2.5% magnesium stearate or stearic acid.

In a further preferred embodiment the following substances and quantities are used:

| | |
|---|---|
| 57.1% Ginkgo biloba dry extract (EGb761^{®}) | (240.000 mg) |
| 40.5% Ethylcellulose | (170.000 mg) |
| 0.5% Colloidal silicon dioxide | ( 2.000 mg) |
| 1.9% Magnesium stearate | ( 8.000 mg) |

The function of these components is the following: Ginkgo biloba dry extract-active principle; ethylcellulose - dry binding agent for direct compression, forming the polymeric matrix producing the controlled release effect; colloidal silicon dioxide - glidant, improves the flowability of the mixture for compression; and magnesium stearate/stearic acid - lubricant, reduces the friction between the mixture for compressing the tablets and the compression tooling.

Ginkgo biloba dry extract (EGb761^{®}) has, due to its herbal origin and the process for obtaining it (extraction with polar solvents), a good water solubility; as fine powder it can be mixed with the ethylcellulose polymer which is practically non-water-soluble; the mixture of these two components can be compressed forming a controlled release matrix. The result is a tablet with good mechanical stability, good appearance and smooth surface, with a high content of Ginkgo biloba extract, incorporated in a polymeric matrix, insoluble in water.

The procedure includes the following stages:
1. Mixing the Ginkgo biloba dry extract (EGb761) with ethylcellulose and silicon dioxide, using a container mixer.
2. Lubricating the previous homogenous mixture by mixing with a part of magnesium stearate.
3. Granulating the lubricated mixture, compacting and dry grinding, using a roller compactor.
4. Sieving and lubricating the resulting powder by mixing with the other part of magnesium stearate, using again a container mixer; and
5. Compressing the lubricated powder into tablets with a hardness of 7 kp or more, using a tabletting machine.

The tablet is optionally provided with a suitable coating, for example made of hydroxymethylcellulose, polyethyleneglycol, pigments (such as titanium dioxide, iron oxide) and talcum.

In the aforementioned manner, it is obtained a controlled released tablet with Ginkgo biloba extract as a simple formulation with few ingredients, by using common compendial excipients, with a high content of herbal extract but small dimensions, produced with a low energy process (direct compression) and without the use of solvents, without significant dependency of the release profile on the tablet hardness. The product has an excellent stability inside its primary container and there is no exposure of the active compound, or the excipients to heating processes, or humidification with water or organic solvents which reduces the physicochemical stress for the herbal active.

### Examples

### Example 1: Preparation of controlled release tablets

2.4 kg dry extract from the leaves of Ginkgo biloba (EGb761^{®}) were mixed with 1.7 kg ethylcellulose and with 20 g colloidal silicon dioxide, using a container mixer. The resulting homogenous mixture was lubricated by mixing with 40 g of magnesium stearate. This lubricated mixture was granulated, compacted and dry grinded, using a roller compactor. The resulting powder was sieved and lubricated by mixing with 40 g of magnesium stearate, using again a container mixer. This lubricated powder was compressed into tablets with a weight of 420 mg (containing 240 mg EGb761^{®}) with a hardness of 8 kp, using a tabletting machine.

### Example 2: Drug release profile

The tablets according to example 1 fulfill the requirements of the European Pharmacopeia 6.0, 2.9.3 with respect to the definition of a prolonged release dosage form by displaying the release profile described in the following table:

**Table 1: Drug release profile of flavonglycosides from Ginkgo biloba extract determined according to European Pharmacopeia 6.0 method 2.9.3 for prolonged release dosage forms. Batch number of the tablets: 060915**

| Time [min] | Drug released [%] |
|---|---|
| 60 | 22.8 |
| 180 | 40.9 |
| 360 | 62.5 |

## Claims

1. Controlled release tablet, **characterized in that** it consists of: a mixture of 52% - 60% dry extract from the leaves of Ginkgo biloba in a polymeric matrix of 37% - 45% ethylcellulose, further comprising 0.4% - 1.0% colloidal silicon dioxide and 1.5% - 2.5% magnesium stearate or stearic acid as excipients.

2. Controlled release tablet according to claim 1, **characterized in that** it consists of:
| | |
|---|---|
| 57.1 % Dry extract from the leaves of Ginkgo biloba | (240.000 mg) |
| 40.5% Ethylcellulose | (170.000 mg) |
| 0.5% Colloidal silicon dioxide | ( 2.000 mg) |
| 1.9% Magnesium stearate | ( 8.000 mg) |

3. Controlled release tablet according to claim 1 or 2, wherein the dry extract from the leaves of Ginkgo biloba is **characterized by** the following contents:
• 20 to 30 % by weight flavonoids, selected from the group comprising quercetin, kaempferol and isorhamnetin glycosides,
• 4.5 to 8.5 % by weight terpene lactones and
• less than 10 ppm ginkgolic acids.

4. Controlled release tablet according to claim 3, wherein the dry extract from the leaves of Ginkgo biloba is **characterized by** the following contents:
• 22.0 to 27.0 % by weight flavonoids, selected from the group comprising quercetin, kaempferol and isorhamnetin glycosides,
• 2.8 to 3.4 % by weight ginkgolides A, B and C in total,
• 2.6 to 3.2 % by weight bilobalid and
• 5 ppm ginkgolic acids at the most.

5. Controlled release tablet according to one of the claims 1 to 4, wherein the dry extract from the leaves of Ginkgo biloba is **characterized by** the following contents:
• less than 10 ppm 4'-O-methyl pyridoxine and/or
• less than 20 ppm biflavones selected from the group comprising amentoflavone, bilobetin, ginkgetin, isoginkgetin and sciadopitysin.

6. Procedure for obtaining a controlled release tablet of Gingko biloba according to one of the claims 1 to 5, **characterized in that** it comprises in combination the following stages:
1. Mixing the dry extract from the leaves of Ginkgo biloba with ethylcellulose and silicon dioxide, using a container mixer.
2. Lubricating the previous homogenous mixture, mixing with a part of magnesium stearate.
3. Granulating the lubricated mixture, compacting and dry grinding, using a roller compactor.
4. Sieving and lubricating the resulting powder, mixing with the other part of magnesium stearate, using again a container mixer; and
5. Compressing the lubricated powder into tablets with a hardness of 7 kp or more, using a tabletting machine.

7. Tablet or procedure according to one of the claims 1 to 6, wherein the controlled release tablet is a prolonged release tablet.

## Patentansprüche

1. Retard-Tablette, **dadurch gekennzeichnet, dass** sie besteht aus: einem Gemisch von 52% - 60% Trockenextrakt von Ginkgo-biloba-Blättern in einer Polymermatrix von 37% - 45% Ethylcellulose, außerdem 0,4% - 1,0% kolloidales Siliciumdioxid und 1,5% - 2,5% Magnesiumstearat oder Stearinsäure als Exzipientien umfassend.

2. Retard-Tablette gemäß Anspruch 1, **dadurch gekennzeichnet, dass** sie besteht aus:
| | |
|---|---|
| 57,1% Trockenextrakt von Ginkgo-biloba-Blättern | (240,000 mg) |
| 40,5% Ethylcellulose | (170,000 mg) |
| 0,5% kolloidalem Siliciumdioxid | ( 2,000 mg) |
| 1,9% Magnesiumstearat | ( 8,000 mg) |

3. Retard-Tablette gemäß Anspruch 1 oder 2, wobei der Trockenextrakt von Ginkgo-biloba-Blättern durch die folgenden Gehalte gekennzeichnet ist:
• 20 bis 30 Gewichts-% Flavonoide, ausgewählt aus der Gruppe, umfassend Quercetin-, Kaempferol- und Isorhamnetinglycoside,
• 4,5 bis 8,5 Gewichts-% Terpenlactone und
• weniger als 10 ppm Ginkgolsäuren.

4. Retard-Tablette gemäß Anspruch 3, wobei der Trockenextrakt aus Ginkgo-biloba-Blättern durch die folgenden Gehalte gekennzeichnet ist:
• 22,0 bis 27,0 Gewichts-% Flavonoide, ausgewählt aus der Gruppe, umfassend Quercetin-, Kaempferol- und Isorhamnetinglycoside,
• 2,8 bis 3,4 Gewichts-% Ginkgolide A, B und C insgesamt,
• 2,6 bis 3,2 Gewichts-% Bilobalid und
• höchstens 5 ppm Ginkgolsäuren.

5. Retard-Tablette gemäß einem der Ansprüche 1 bis 4, wobei der Trockenextrakt von den Ginkgo-biloba-Blättern durch die folgenden Gehalte gekennzeichnet ist:
• weniger als 10 ppm 4'-O-Methylpyridoxin und/oder
• weniger als 20 ppm Biflavone, ausgewählt aus der Gruppe, umfassend Amentoflavol, Bilobetin, Ginkgetin, Isoginkgetin und Sciadopitysin.

6. Verfahren zur Herstellung einer Gingko-biloba-Retard-Tablette gemäß einem der Ansprüche 1 bis 5, **dadurch gekennzeichnet, dass** es die folgenden Stufen in Kombination umfasst:
1. Mischen des Trockenextraktes von den Ginkgo-biloba-Blättern mit Ethylcellulose und Siliciumdioxid unter Verwendung eines Containermischers.
2. Schmieren des vorherigen homogenen Gemisches, Mischen mit einem Teil von Magnesiumstearat.
3. Granulieren des geschmierten Gemisches, Kompaktieren und Trockenzerkleinern, wobei eine Walzenpresse verwendet wird.
4. Sieben und Schmieren des resultierenden Pulvers, Mischen mit dem anderen Teil an Magnesiumstearat, wobei wiederum ein Containermischer verwendet wird; und
5. Verpressen des geschmierten Pulvers in Tabletten mit einer Härte von 7 kp oder mehr, wobei eine Tablettiermaschine verwendet wird.

7. Tablette oder Verfahren gemäß einem der Ansprüche 1 bis 6, wobei die Retard-Tablette eine Tablette mit verlängerter Freisetzung ist.

## Revendications

1. Comprimé à libération contrôlée, **caractérisé en ce qu'**il consiste en un mélange de 52% à 60% d'extrait sec de feuilles de ginkgo biloba dans une matrice polymérique de 37% - 45% d'éthylcellulose,
comprenant en outre de 0,4% à 1,0% de dioxyde de silicium colloïdal et de 1,5% à 2,5% de stéarate de magnésium ou de l'acide stéarique comme excipients.

2. Comprimé à libération contrôlée selon la revendication 1, **caractérisé en ce qu'**il consiste en:
57,1% d'extrait sec de feuilles de ginkgo biloba (240,000 mg)
40,5% d'éthylcellulose (170,000 mg),
0,5% de dioxyde de silicium colloïdal (2,000 mg),
1,9% de stéarate de magnésium (8,000 mg).

3. Comprimé à libération contrôlée selon la revendication 1 ou 2, ledit extrait sec de feuilles de gingko biloba étant **caractérisé par** les teneurs suivantes:
• de 20 à 30% en poids de flavonoïdes, choisis dans le groupe comprenant des glycosides de quercétine, de kaempférol et d'isorhamnétine;
• de 4,5 à 8,5% en poids de lactones terpéniques et
• moins de 10 ppm d'acides ginkgoliques.

4. Comprimé à libération contrôlée selon la revendication 3, ledit extrait sec des feuilles de ginkgo biloba étant **caractérisé par** les teneurs suivantes:
• de 22,0 à 27,0% en poids de flavonoïdes, choisis dans le groupe comprenant des glycosides de quercétine, de kaempférol et d'isorhamnétine,
• de 2,8 à 3,4% en poids au total de ginkgolides A, B et C,
• de 2,6 à 3,2% en poids de bilobalide, et
• au plus 5 ppm d'acides ginkgoliques.

5. Comprimé à libération contrôlée selon l'une quelconque des revendications 1 à 4, ledit extrait sec de feuilles de ginkgo biloba étant **caractérisé par** les teneurs suivantes:
• moins de 10 ppm de 4'-O-méthyl pyridoxine et/ou
• moins de 20 ppm de biflavones, choisis dans le groupe comprenant l'amentoflavone, la bilobetine, la ginkgetine, l'isoginkgetine et la sciadopitysine.

6. Procédé d'obtention d'une comprimé à libération contrôlée de ginkgo biloba selon l'une quelconque des revendications 1 à 5, **caractérisé en ce qu'**il comprend en combinaison les étapes suivantes:
1. Mélanger l'extrait sec de feuilles de ginkgo biloba avec de l'éthylcellulose et du dioxyde de silicium, en utilisant un mélangeur à récipient.
2. Lubrifier le mélange homogène précédent en mélangeant avec une partie de stéarate de magnésium.
3. Granuler le mélange lubrifié, compacter et meuler à sec en utilisant un compacteur à rouleaux.
4. Tamiser et lubrifier la poudre obtenue, mélanger avec l'autre partie de stéarate de magnésium, en utilisant de nouveau un mélangeur à récipient.
5. Comprimer la poudre lubrifiée en comprimés avec une dureté de 7 kp ou plus en utilisant.une machine de production de comprimés.

7. Comprimé ou procédé selon une des revendications 1 à 6, le comprimé à libération étant un comprimé à libération prolongée.
